# EUROPEAN PATENT SPECIFICATION

(11) **EP 3 006 937 B1**
(45) Date of publication and mention of the grant of the patent: **05.12.2018**
(21) Application number: 13886328.7
(22) Date of filing: 19.06.2013
(51) Int. Cl.: G01N 33/52, G01N 33/84, G01N 21/80, G01N 31/22

(54) **PORTABLE PAPER CHIP CAPABLE OF VISUALLY DETECTING CHLORINE ION CONTENT IN SWEAT**
TRAGBARES PAPIERBLATT ZUR SICHTERFASSUNG DES CHLORIONENGEHALTS IN SCHWEISS
MORCEAU DE PAPIER PORTABLE PERMETTANT DE DÉTECTER VISUELLEMENT LA TENEUR EN IONS CHLORE DE LA TRANSPIRATION

(30) Priority: 08.06.2013 CN 201310228132
(43) Date of publication of application: 13.04.2016
(73) Proprietor: Institute of Basic Medical Sciences, Chinese Academy of Medical Sciences, Beijing 100005 (CN)
(72) Inventor: ZHENG, Zhi, Beijing 100005 (CN); MU, Xuan, Beijing 100005 (CN)
(74) Representative: LKGLOBAL Lorenz & Kopf PartG mbB Patentanwälte
(86) International application number: PCT/CN2013/077471
(87) International publication number: WO 2014/194537

(56) References cited:
- CN-A- 1 100 809
- CN-A- 1 615 441
- GB-A- 2 495 851
- US-A- 3 449 080
- US-A- 3 449 080
- US-A- 4 211 532
- US-A- 4 444 193
- US-A- 5 691 205
- US-A- 6 042 543
- US-A- 6 042 543
- US-A1- 2009 157 023

## Description

### FIELD OF THE INVENTION

The present invention relates to a portable paper-based device as specified in claim 1, which is capable of *in-situ* detecting of chlorine ion content in sweat. Such device can *inter alia* be used for monitoring the physiological status and preliminarily screening of cystic fibrosis (a hereditary disease).

### BACKGROUND

A portable device capable of visually detecting body fluid is one of the most important development directions of personalized medicine in the future. A portable device may allow clinical detection performed at home and in public places, and visually detected results may rapidly provide physiological and pathological information in the body fluid, which greatly improves medical diagnostic efficiency. Sweat is a major form of body fluids, containing a variety of compounds such as salt, sugar, and organic acid. The main features of sweat are as follows: (1) it is directly excreted from the body, so its collection for detection can be noninvasive, with very low infection risk; (2) it is convenient for obtaining sweat since perspiration can be stimulated by exercising, heat and compounds; and (3) the chemical components of sweat are correlated with specific physiological and pathological conditions. For example, when the body is dehydrated, the chloride ion concentration in sweat will be significantly increased. Dehydrated state of the body can be learned by monitoring chloride ion concentration in sweat so as to promote effective re-hydration and prevent chronic and acute diseases caused by dehydration. Additionally, one of the trans-membrane transporters in patients suffering from cystic fibrosis is altered so that chloride ions cannot be recovered from sweat, thereby also leading to an increase in chloride ion concentration in sweat. The method of detecting chloride ion concentration in sweat has become a primary means and gold standard for diagnosis of cystic fibrosis.

Although the detection of chloride ion in sweat is of great clinical significance and practical value, the corresponding detection technology and device are still inadequate; especially the ability of portable and *in-situ* detection is still needed. The procedure in a conventional method for detecting chloride ion in sweat is typically as follows: (1) stimulating sweat excretion by subcutaneously introducing pilocarpine ions; (2) collecting the excreted sweat by using a pre-weighted, dried gauze; (3) weighting the gauze after absorbing the sweat, and calculating the volume of the excreted sweat; and (4) determining the ion concentration in the elution solution from the gauze by using an atomic spectrophotometer. In addition to gauze, the liquid storage bag and cotton cloth patch may also be used to collect sweat, as described in, for example, "Sweat mineral loss from whole body, patch and arm bag in white and black girls". C. Palacios, K et al. Nutrition Research 23, 401 (Mar, 2003).

Currently, the most commonly used instrument commercially available is Macroduct System developed by Wescor Inc., USA(Wescor Inc., http://www.wescor.com/ biomedical/cysticfibrosis/macroduct.html and M. R. Ely et al., Evaluation of the Megaduct sweat collector for mineral analysis. Physiological Measurement 33, 385 (Mar, 2012)). The System is also approved by American Cystic Fibrosis Association and can be used for diagnosing cystic fibrosis diseases. However, the biggest problem of these methods is in that the step for collecting sweat is relatively separated from that for detecting sweat, so that a step for transferring sweat is needed between collection and detection steps, and it is not only impossible to obtain in-situ real time detection results, but also is error prone. The study by M. Constantinescu and B. C. Hilman, "The sweat test for quantitation of electrolytes - A challenge in precision". Laboratory Medicine 27, 472 (Jul, 1996) reported that per minute 2% of the volume of sweat collected on filter paper and cotton would be lost due to evaporation. Moreover, Macroduct System is complex in structure, complicated in detection procedures, and also more expensive in price, limiting its further application.

In addition to the above technology with independent collection and detection steps, *in-situ* methods for detecting chloride ion in sweat have also been reported. In some early studies, chloride ion in sweat was detected by utilizing the reaction of silver chromate with chlorine ion to generate silver nitride precipitation (see, J. B. J. S. S. J. Knights Em, Simplified screening test for cystic fibrosis of the pancreas. The Journal of the American Medical Association 169, 1279 (1959); H. Shwachman, N. Gahm, Studies in Cystic Fibrosis of the Pancreas. N. Engl. J. Med. 255, 999 (1956); L. Gluck, A Patch Test For Chloride In Sweat As A Simple Screening Method For Detecting Cystic Fibrosis Of The Pancreas: Preliminary Report. Pediatrics 23, 731 (April 1, 1959)). While this detection is easily performed with straightforward result by placing the sweaty palms on agarose gel and filter paper, the silver chromate is toxic and carcinogenic. American Cystic Fibrosis Association has expressly prohibited the use of silver chloride precipitation-based detection reactions on the skin (see V. A. LeGrys, J. R. Yankaskas, L. M. Quittell, B. C. Marshall, P. J. McGayzel, Diagnostic sweat testing: The cystic fibrosis foundation guidelines. Journal of Pediatrics 151, 85 (Jul, 2007)). Recently, a research group has developed a wearable device for the detection of chloride ion in sweat (see B. Schazmann et al., A wearable electrochemical sensor for the real-time measurement of sweat sodium concentration. Analytical Methods 2, 342 (Apr, 2010)). The device mainly consists of an electronic multimeter and a chloride ion-selective electrode. It requires larger sampling volume (i.e., a lot of sweat), which limits the applications. In addition, the size and the weight of the device seriously affect the portability of the device.

Various test devices for the *in-situ* detection of chlorine ion content in sweat are also disclosed in patent documents US 3,449,080; US 6,042,543; GB 2 495 851; CN 1 100 809; and US 2009/0157023. All these devices have a single layer of paper comprising an anion exchange material and a pH indicator.

### SUMMARY OF THE INVENTION

The present invention provides a portable paper-based device for the rapid in-situ detection of chlorine ion content in sweat, comprising (i) a paper capable of generating color or fluorescence changes according to pH; and (ii) an anion exchange paper being arranged on the lower surface of said paper to form a double-layer structure, wherein the anion exchange paper is a paper modified with anion exchange groups, the latter being selected from diethylaminoethyl groups or quaternary ammonium groups.

In particular embodiments, the portable paper-based device of the present invention further comprises (iii) an adhesive sticker being arranged on the upper surface of the paper capable of generating color or fluorescence changes according to pH, and (iv) a paper capable of characterizing the volume of absorbed liquid arranged on the lower surface of the adhesive sticker, wherein the paper capable of characterizing the volume of absorbed liquid have a liquid inlet end. Preferably, the portable paper-based device further comprises (v) a substrate arranged on the lower surface of the portable paper-based device, wherein the anion exchange paper, the paper capable of generating color or fluorescence changes according to pH, and the paper capable of characterizing the volume of absorbed liquid are immobilized between the substrate and the adhesive sticker, and there is a pore on the substrate, in position opposite to the anion exchange paper and the liquid inlet end of the paper capable of characterizing the volume of absorbed liquid.

In particular embodiments, the paper capable of generating color or fluorescence changes according to pH is preferably a pH test paper or a paper infiltrated with pH-responsive fluorescent dye, and the substrate is an impermeable material, preferably an impermeably adhesive tape.

In further particular embodiments, the paper capable of characterizing the volume of absorbed liquid has preferably fluorescent or color markers and indicator scales.

Preferably, the adhesive sticker is transparent, semi-transparent or has a visible window.

In another aspect, the present invention provides the use of the portable paper-based device of the present invention for the non-invasive *in-situ* detection of chlorine ion content in sweat.

In yet another aspect, the present invention provides a non-invasive method for the rapid *in-situ* detection of chlorine ion content in sweat, comprising the steps of: (a) covering the skin surface with the portable paper-based device of the present invention for absorbing sweat by the anion exchange paper; and (b) determining the chlorine ion content in sweat based on the color or fluorescence changes on the paper capable of generating color or fluorescence changes according to pH, and according to the standard curve pre-plotted.

In yet another aspect, the present invention provides a non-invasive method for the rapid *in-situ* detection of chlorine ion content in sweat, comprising the steps of (a) covering the skin surface with the portable paper-based device of the present invention for absorbing sweat by the anion exchange paper and the liquid inlet end of the paper capable of characterizing the volume of absorbed liquid; and (b) determining the volume of the absorbed sweat by the paper capable of characterizing the volume of absorbed liquid, and taking down the portable paper-based device when the volume of the absorbed sweat reaches a determined value; and determining chlorine ion content in sweat based on the color or fluorescence changes on the paper capable of generating color changes according to pH and according to the standard curve pre-plotted.

Preferably, the volume of the absorbed sweat is 0.1 µl to 10 µl, more preferably 2 µl.

Drawings and specific embodiments described herein are provided to illustrate certain principles of the present invention.

The beneficial effects of the present invention are as follows:
The paper-based device integrates the functions of sweat collection and detection and the like, avoids the step of sweat transfer, simplifies the procedures, and improves the accuracy of detection.

The paper-based device is capable of *in-situ,* real-time and visually detecting chloride ions in sweat, facilitating to monitor physiological conditions.

The paper-based device is mainly composed of paper, highly portable and low cost, and is very suitable for large-scale screening and daily use.

The paper-based device can be used on the skin due to use of adhesive sticker, which is biologically safe.

### BRIEF DESCRIPTION OF THE DRAWINGS

Hereinafter, with reference to drawings specific embodiments of the present invention are described in detail. It should be understood that the following technical scheme is only for illustrative purposes.

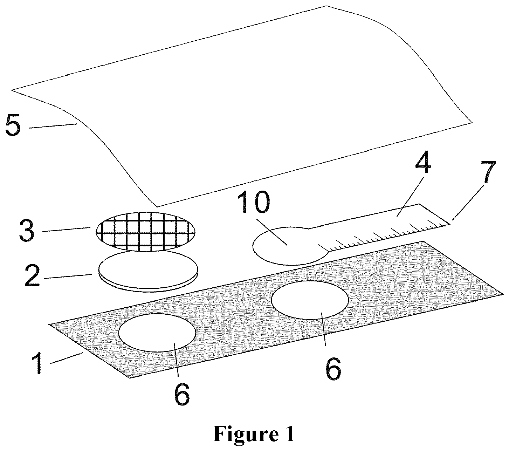

Figure 1 is a schematic view of the portable paper-based device used herein for *in-situ* detection of chloride ions; wherein 1 represents an impermeable material, 2 represents an anion exchange paper, 3 represents a paper generating color or fluorescence changes according to pH, 4 represents a paper characterizing the volume of absorbed liquid, 5 represents adhesive sticker, 6 represents empty pore, 7 represents scales, and 10 represents the liquid inlet end of the paper characterizing the volume of absorbed liquid.

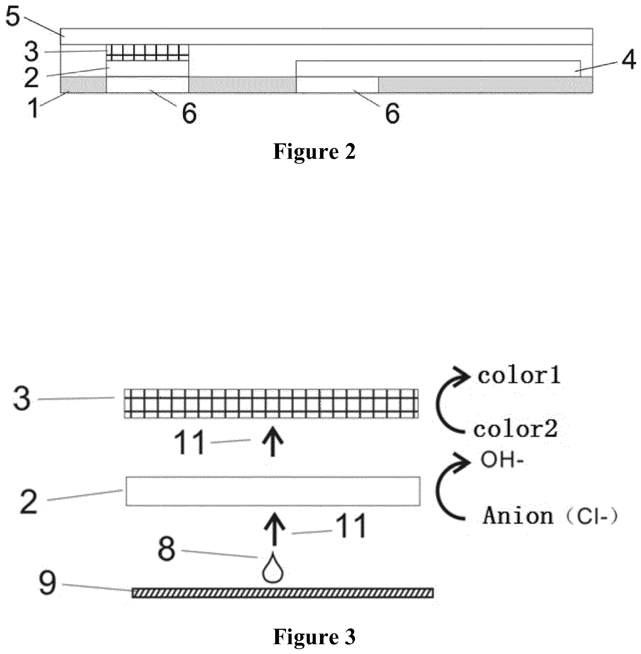

Figure 2 is a cross-sectional schematic view of the portable paper-based device used herein for *in-situ* detection of chloride ions content; wherein 1 represents an impermeable material, 2 represents an anion exchange paper, 3 represents a paper generating color or fluorescence changes according to pH, 4 represents a paper characterizing the volume of absorbed liquid, 5 represents adhesive sticker, 6 represents empty pore, and 7 represents scales.
Figure 3 is a schematic view of the mechanism underlying the detection of chloride ions, wherein 2 represents an anion exchange paper, 3 represents a paper generating color or fluorescence changes according to pH, 8 represents sweat, 9 represents skin from healthy persons or patients suffering from cystic fibrosis; and the arrow of 11 represents the direction of liquid flow.

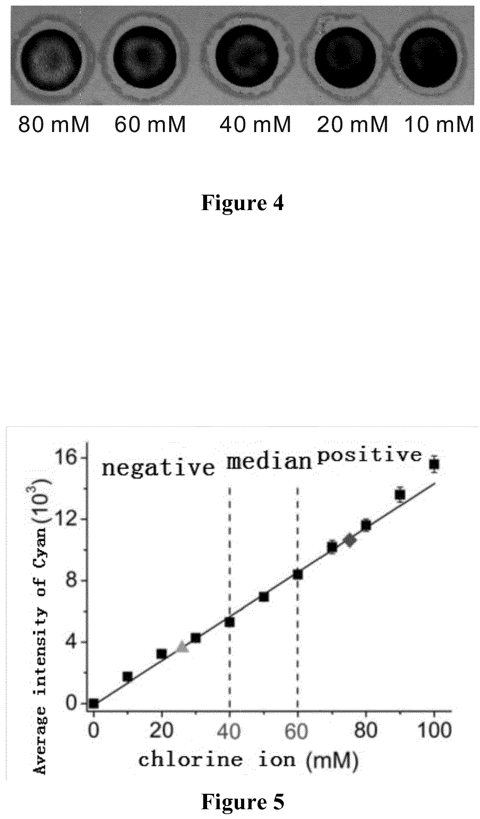

Figure 4 shows color changes corresponding to various chloride ion concentrations. Five round pores are corresponding to the five detection region. The figures below represent chloride ion concentrations, and the color channel represents Cyan in CMYK color space.
Figure 5 shows a standard curve for detection of 0 to 100mmol chloride ion, indicating the test results of healthy people (triangles) and patients suffering from cystic fibrosis (diamonds).

It should be understood that the appended drawings do not proportionally show the basic principles of the present invention. They illustrate various features with somewhat simplified painting. The specific design features disclosed herein, including, for example, specific dimensions, orientations, locations, and shapes are determined in part by the particular application and environment.

In these drawings, ligands throughout the drawings are referred to the same or similar parts of the present invention.

The following specific embodiments are only intended for illustration of the present invention.

### DETAILED DECRIPTION OF THE INVENTIION

The present invention relates to a paper-based device as specified in clam 1, made of an anion exchange paper and a paper capable of generating color or fluorescence changes according to pH, the paper-based device is capable of visually detecting chlorine ion.

In the present invention, the paper is preferably made of multilayer film composed of three-dimensional cellulose fibers, being hydrophilic. Wherein the anion exchange paper is a paper modified with anion exchange groups (e.g., diethylaminoethyl and quaternary ammonium groups), it is capable of absorbing anions in liquid which then exchanged for hydroxide ions. Wherein the paper capable of generating color changes according to pH is a pH test paper (e.g. extensive and precise pH test paper), it can reflect changes in pH by color changes. The paper capable of generating fluorescence changes according to pH is replaced with the paper modified with pH-sensitive fluorescent dyes and quantum dots. Preferably, the paper-based device of the present invention further comprises a paper characterizing the volume of absorbed liquid, which has scales and fluorescein or dye color markers. When liquid is absorbed through the liquid inlet end of the paper, markers will indicate the front end of liquid, thereby indicating the volume of liquid absorbed by the paper. More preferably, the paper-based device of the present invention has a transparent and adhesive sticker, such as 3M tegaderm.

The functional paper is cut, three-dimensionally assembled and integrated by using the microfluidic paper-chip technology, so as to build a complete microfluidic paper-based device, and to achieve function for visual detection of chloride ions. Sweat on the skin first passes through the anion exchange paper, where the anions are exchanged for hydroxide ions. The volume of sweat is estimated with the paper indicating the volume of liquid. It should be noted that chloride ion is the main anion in sweat. Its content is thousands of times higher than that of sulfate anion, the second major anion. After anion is exchanged into hydroxyl ion, the pH value of sweat will be increased accordingly; the range of changes is correlated with the hydroxide. After passing through the anion exchange paper, sweat will be contacted with the pH paper. Color change on pH paper may indicate changes in the pH value. Similarly, pH responsive fluorescent materials may also be used to indicate the change in pH value. Changes in color or fluorescence are collected by a CCD or a smart phone, via image processing, Red channel in RGB color space or Cyan channel in CMYK color space is separated. The concentration of chloride in sweat is indicated by using single-channel color gradation.

### Example 1:

Step One: The paper structure was made according to design size, as shown in Figure 1. 6 represents a circular pore with a diameter of 1-3 mm. 2 represents the anion exchange paper, Whatman DE81 or Pall Mustang, with a diameter of 2-4 mm, slightly larger than that of 6. 3 represents a pH test paper or a paper modified with pH-sensitive quantum dots (fluorescent dye), the diameter of which is 3-5 mm, slightly larger than that of 2. The diameter of the round end of 4 is 2-4 mm, slightly larger than that of 6. The length of 4 is 2 cm. The length of 1 is 3-5 cm, and the width of 1 is 1-3 cm. 7 is produced by the inkjet printer. 5 represents 3M Tegaderm adhesive sticker, the size of which is slightly larger than that of 1, with the length of 4-6 cm, and the width of 2-4 cm.

Step Two: 1 to 5 were assembled together as designed so as to give a paper-based device. 5 is sticky, and the positions of other components were immobilized by closely binding 5 with the 1. The cross-section of the paper-based device after assembly is shown in Figure 2.

Step Three: Gradient concentrations of sodium chloride solution, 10 mM - 80 mM, were prepared.

Step Four: 1 µl sodium chloride solution was dropped onto 6 of 1, so that liquid flows onto 2 and 3; chlorine ions were exchanged into hydroxide ions, the pH value of liquid was increased, and the color of pH paper was changed, see Figure 3.

Step Five: The color change on the pH test paper was captured with CCD or smart phone, see Figure 4. Shooting background is generally plain blue, and shooting light was evenly distributed.

Step Six: Color information on a single-channel, such as R channel in RGB and C channel in CMYK, was separated with software (such as NIH ImageJ or Adobe Photoshop), and was associated with the concentration of chlorine ion. A standard curve of chloride ion concentration was established.

### Example 2:

Step One: The paper structure was made according to design size, as shown in Figure 1. The paper structure was made according to design size, and the paper structure was assembled correspondingly. The diameter of 6 is 1-3 mm. The diameter of 2 is 2-4 mm, slightly larger than that of 6. The diameter of 3 is 3-5 mm, slightly larger than that of 2. The diameter of the round end of 4 is 2-4 mm, slightly larger than that of 6. The length of 4 is 2 cm. The length of 1 is 3-5 cm, and the width of 1 is 1-3 cm. The size of 5 is slightly larger than that of 1, with the length of 4-6 cm, and the width of 2-4 cm.

Step Two: 1 to 5 were assembled together as designed so as to give a paper-based device. 5 is sticky. The positions of other components were immobilized by closely binding 5 with the 1. The cross-section of the paper-based device after assembly is shown in Figure 2.

Step Three: Gradient concentrations of sodium chloride solution, 0 mM - 100 mM, were prepared.

Step Four: Detected sodium chloride solution and established a standard curve. The paper-based device was placed on 9 and allowed sweat flow into the paper-based device.

Step Five: The paper-based device was removed as the scale indicating 2ul. Alternatively, image information was directly acquired with CCD or smart phone.

Step Six: Color information on a single-channel, such as Red channel in RGB color space and Cyan channel in CMYK color space, was separated with software (such as app of smartphone, NIH ImageJ or Adobe Photoshop). The concentration of chloride ion was obtained by using the standard curve.

## Claims

1. A portable paper-based device for the rapid *in-situ* detection of chlorine ion content in sweat, comprising:
- a paper capable of generating color or fluorescence changes according to pH; and
- an anion exchange paper, being arranged on the lower surface of said paper to form a double-layer structure;
wherein the anion exchange paper is a paper modified with anion exchange groups, the latter being selected from diethylaminoethyl groups or quaternary ammonium groups.

2. The portable paper-based device according to claim 1, further comprising:
- an adhesive sticker, being arranged on the upper surface of the paper capable of generating color or fluorescence changes according to pH; and
- a paper capable of characterizing the volume of absorbed liquid arranged on the lower surface of the adhesive sticker;
wherein the paper capable of characterizing the volume of absorbed liquid have a liquid inlet end.

3. The portable paper-based device according to claim 2, further comprising:
- a substrate, being arranged on the lower surface of the portable paper-based device;
wherein the anion exchange paper, the paper capable of generating color or fluorescence changes according to pH, and the paper capable of characterizing the volume of absorbed liquid are immobilized between the substrate and the adhesive sticker; and wherein a pore is arranged on the substrate, in position opposite to the anion exchange paper and the liquid inlet end of the paper capable of characterizing the volume of absorbed liquid.

4. The portable paper-based device according to any one of claims 1 to 3, wherein the paper capable of generating color or fluorescence changes according to pH is a pH test paper or a paper infiltrated with pH-responsive fluorescent dye, and the substrate is an impermeable material.

5. The portable paper-based device according to claim 4, wherein the impermeable material is an impermeably adhesive tape.

6. The portable paper-based device according to any one of claims 1 to 5, wherein the paper capable of characterizing the volume of absorbed liquid has fluorescent or color markers and indicator scales.

7. The portable paper-based device according to any one of claims 1 to 6, wherein the adhesive sticker is transparent, semi transparent or has a visible window.

8. Use of the portable paper-based device according to any one of claims 1 to 7 for the non-invasive *in-situ* detection of chlorine ion content in sweat.

9. A non-invasive method for the rapid *in-situ* detection of chlorine ion content in sweat, comprising the steps of:
(a) covering the skin surface with the portable paper-based device according to any one of claims 1 to 7 for absorbing sweat by the anion exchange paper;
(b) determining the chlorine ion content in sweat based on the color or fluorescence changes on the paper capable of generating color or fluorescence changes according to pH, and according to the standard curve pre-plotted.

10. A non-invasive method for the rapid *in-situ* detection of chlorine ion content in sweat, comprising the steps of:
(a) covering the skin surface with the portable paper-based device according to any one of claims 2 to 7 for absorbing sweat by the anion exchange paper and the liquid inlet end of the paper capable of characterizing the volume of absorbed liquid;
(b) determining the volume of the absorbed sweat by the paper capable of characterizing the volume of absorbed liquid, and taking down the portable paper-based device when the volume of the absorbed sweat reaches a determined value; and determining chlorine ion content in sweat based on the color or fluorescence changes on the paper capable of generating color changes according to pH and according to the standard curve pre-plotted.

11. The method according to claim 9, wherein the volume of the absorbed sweat is 0.1 µl to 10 µl, and particularly is 2 µl.

## Patentansprüche

1. Eine transportable, auf Papier basierende Vorrichtung zur schnellen *in-situ* Detektion des Gehalts an Chlorionen in Schweiß, die aufweist:
- ein Papier, das zur Erzeugung von Farb- oder Fluoreszenzänderungen aufgrund des pHs in der Lage ist; und
- ein Anionenaustauschpapier, das auf der Unterseite des besagten Papiers angeordnet ist, um eine Zweischichtstruktur zu bilden;
wobei das Anionenaustauschpapier ein mit Anionenaustauschgruppen modifiziertes Papier ist, und letztere aus Diethylaminoethyl-Gruppen und quarternären Ammoniumgruppen ausgewählt werden.

2. Die transportable, auf Papier basierende Vorrichtung gemäß Anspruch 1, die ferner aufweist:
- ein Klebeetikett, das auf der Oberseite des Papiers angeordnet ist, welches zur Erzeugung von Farb- oder Fluoreszenzänderungen aufgrund des pHs in der Lage ist; und
- ein Papier, das zur Bestimmung des Volumens an absorbierter Flüssigkeit in der Lage ist, angeordnet auf der Unterseite des Klebeetiketts;
wobei das Papier, das zur Bestimmung des Volumens an absorbierter Flüssigkeit in der Lage ist, ein Ende zum Flüssigkeitseingang aufweist.

3. Die transportable, auf Papier basierende Vorrichtung gemäß Anspruch 2, die ferner aufweist:
- ein Substrat, das auf der Unterseite der transportablen, auf Papier basierenden Vorrichtung angeordnet ist;
wobei das Anionenaustauschpapier, das Papier, welches zur Erzeugung von Farb- oder Fluoreszenzänderungen aufgrund des pHs in der Lage ist, und das Papier, welches zur Bestimmung des Volumens an absorbierter Flüssigkeit in der Lage ist, zwischen dem Substrat und dem Klebeetikett immobilisiert sind, und wobei auf dem Substrat eine Pore angeordnet ist, deren Position gegenüber dem Anionenaustauschpapier und dem Ende zum Flüssigkeitseingang des Papier liegt.

4. Die transportable, auf Papier basierende Vorrichtung gemäß einem der Ansprüche 1 bis 3, wobei das Papier, das zur Erzeugung von Farb- oder Fluoreszenzänderungen aufgrund des pHs in der Lage ist, ein pH-Testpapier ist oder ein Papier, das mit einem auf Fluoreszenz reagierenden Farbstoff infiltriert ist, und wobei das Substrat ein impermeables Material ist.

5. Die transportable, auf Papier basierende Vorrichtung gemäß Anspruch 4, wobei das impermeable Material ein impermeables Klebeband ist.

6. Die transportable, auf Papier basierende Vorrichtung gemäß einem der Ansprüche 1 bis 5, wobei das Papier, das zur Bestimmung des Volumens an absorbierter Flüssigkeit in der Lage ist, Fluoreszenz- oder Farbmarker und Indikatorskalen aufweist.

7. Die transportable, auf Papier basierende Vorrichtung gemäß einem der Ansprüche 1 bis 6, wobei das Klebeetikett transparent oder semitransparent ist oder ein Sichtfenster aufweist

8. Verwendung der transportablen, auf Papier basierenden Vorrichtung gemäß einem der Ansprüche 1 bis 7 zur nicht-invasiven *in-situ* Detektion des Gehalts an Chlorionen in Schweiß

9. Nicht-invasives Verfahren zur schnellen *in-situ* Detektion des Gehalts an Chlorionen in Schweiß, welches die Schritte aufweist:
(a) Bedecken der Hautoberfläche mit der transportablen, auf Papier basierenden Vorrichtung gemäß einem der Ansprüche 1 bis 7 zur Absorption von Schweiß mit Hilfe des Anionenaustauschpapiers;
(b) Bestimmen des Gehalts an Chlorionen in Schweiß basierend auf Farb- oder Fluoreszenzänderungen auf dem Papier, das zur Erzeugung von Farb- oder Fluoreszenzänderungen aufgrund des pHs in der Lage ist, und aufgrund einer vorher erstellten Standardkurve.

10. Nicht-invasives Verfahren zur schnellen *in-situ* Detektion des Gehalts an Chlorionen in Schweiß, das die Schritte aufweist:
(a) Bedecken der Hautoberfläche mit der transportablen, auf Papier basierenden Vorrichtung gemäß einem der Ansprüche 2 bis 7 zur Absorption von Schweiß mit Hilfe des Anionenaustauschpapiers und des Endes zum Flüssigkeitseingang des Papiers, das zur Bestimmung des Volumens an absorbierter Flüssigkeit in der Lage ist;
(b) Bestimmern des Volumens an absorbiertem Schweiß durch das Papier, das zur Bestimmung des Volumens an absorbierter Flüssigkeit in der Lage ist, und Abnehmen der transportablen, auf Papier basierenden Vorrichtung, wenn das Volumen des absorbiertes Schweißes einen vorbestimmten Wert erreicht; und Bestimmen des Gehalts an Chlorionen in Schweiß basierend auf Farb- oder Fluoreszenzänderungen auf dem Papier, das zur Erzeugung von Farb- oder Fluoreszenzänderungen aufgrund des pHs in der Lage ist, und aufgrund einer vorher erstellten Standardkurve.

11. Verfahren gemäß Anspruch 9, wobei das Volumen des adsorbierten Schweißes zwischen 0.1 µl und 10 µl liegt, und insbesondere 2 µl beträgt.

## Revendications

1. Dispositif à base de papier portable pour la détection *in situ* rapide de la teneur en ions chlore de la sueur, comprenant:
- un papier capable de générer des changements de couleur ou de fluorescence en fonction du pH; et
- un papier échangeur d'anions, disposé sur la surface inférieure dudit papier pour former une structure à double couche;
dans lequel le papier échangeur d'anions est un papier modifié avec des groupes échangeurs d'anions, ces derniers étant choisis parmi les groupes diéthylaminoéthyle ou les groupes ammonium quaternaire.

2. Dispositif à base de papier portable selon la revendication 1, comprenant en outre:
- un étiquette adhésif, disposé sur la surface supérieure du papier capable de générer des changements de couleur ou de fluorescence en fonction du pH; et
- un papier capable de caractériser le volume de liquide absorbé disposé sur la surface inférieure de l'étiquette adhésif;
dans lequel le papier capable de caractériser le volume de liquide absorbé présente une extrémité d'entrée de liquide.

3. Dispositif à base de papier portable selon la revendication 2, comprenant en outre:
- un substrat, disposé sur la surface inférieure du dispositif à papier portable;
dans lequel le papier échangeur d'anions, le papier capable de générer de la couleur ou de la fluorescence change selon le pH, et le papier capable de caractériser le volume de liquide absorbé est immobilisé entre le substrat et l'étiquette adhésive; et dans lequel un pore est disposé sur le substrat, en position opposée au papier échangeur d'anions et à l'extrémité d'entrée de liquide du papier capable de caractériser le volume de liquide absorbé.

4. Dispositif à papier portable selon l'une quelconque des revendications 1 à 3, dans lequel le papier capable de générer des changements de couleur ou de fluorescence en fonction du pH est un papier test de pH ou un papier infiltré avec un colorant fluorescent sensible au pH et le substrat est un matériau imperméable.

5. Dispositif à base de papier portable selon la revendication 4, dans lequel le matériau imperméable est un ruban adhésif imperméable.

6. Dispositif à papier portable selon l'une quelconque des revendications 1 à 5, dans lequel le papier capable de caractériser le volume de liquide absorbé présente des marqueurs fluorescents ou de couleur et des échelles d'indication.

7. Dispositif à base de papier portable selon l'une quelconque des revendications 1 à 6, dans lequel la vignette adhésive est transparente, semi transparente ou présente une fenêtre visible.

8. Utilisation du dispositif à papier portable selon l'une quelconque des revendications 1 à 7 pour la non invasif détection *in situ* de la teneur en ions chlore dans la sueur.

9. Procédé non invasif pour la détection rapide *in situ* de la teneur en ions chlore de la sueur, comprenant les étapes consistant à:
(a) recouvrir la surface de la peau avec le dispositif à base de papier portable selon l'une quelconque des revendications 1 à 7 pour absorber la sueur par le papier échangeur d'anions;
(b) déterminer la teneur en ions chlore dans la sueur en fonction des changements de couleur ou de fluorescence sur le papier capable de générer des changements de couleur ou de fluorescence en fonction du pH et selon la courbe standard pré-représentée.

10. Procédé non invasif pour la détection rapide *in situ* de la teneur en ions chlore de la sueur, comprenant les étapes consistant à:
(a) recouvrir la surface de peau avec le dispositif à papier portable selon l'une quelconque des revendications 2 à 7 pour absorber la sueur par le papier échangeur d'anions et l'extrémité d'entrée de liquide du papier capable de caractériser le volume de liquide absorbé;
(b) déterminer le volume de la sueur absorbée par le papier capable de caractériser le volume de liquide absorbé et de démonter le dispositif portable à base de papier lorsque le volume de la sueur absorbée atteint une valeur déterminée; et déterminer la teneur en ions chlore dans la sueur en fonction des changements de couleur ou de fluorescence sur le papier capable de générer des changements de couleur en fonction du pH et conformément à la courbe standard pré-représentée.

11. Procédé selon la revendication 9, dans lequel le volume de la sueur absorbée est de 0,1 µl à 10 µl, et en particulier est de 2 µl.
